(19) Europäisches Patentamt
European Patent Office
Office européen des brevets

(11) EP 1 994 959 A1

(12) **EUROPÄISCHE PATENTANMELDUNG**

(43) Veröffentlichungstag:
26.11.2008 Patentblatt 2008/48

(51) Int Cl.:
*A61N 5/10* *(2006.01)*

(21) Anmeldenummer: 08103700.4

(22) Anmeldetag: 24.04.2008

(84) Benannte Vertragsstaaten:
AT BE BG CH CY CZ DE DK EE ES FI FR GB GR HR HU IE IS IT LI LT LU LV MC MT NL NO PL PT RO SE SI SK TR
Benannte Erstreckungsstaaten:
AL BA MK RS

(30) Priorität: 23.05.2007 DE 102007023919

(71) Anmelder: SIEMENS AKTIENGESELLSCHAFT
80333 München (DE)

(72) Erfinder:
• Löseken, Jochen Miguel
95445 Bayreuth (DE)
• Beyer, Lukas
20539 Hamburg (DE)
• Heinl, Dieter
92681 Erbendorf (DE)

(54) **Verfahren und Vorrichtung zur automatischen Positionskorrektur einer Patientenliege für eine zielgerichtete Bestrahlung eines Patienten**

(57) Die Erfindung betrifft ein Verfahren und eine Vorrichtung zur automatischen Positionskorrektur einer Patientenliege für eine zielgerichtete Bestrahlung eines Patienten. Dabei wird die Positionskorrektur nach Maßgabe von Patientendaten vorgenommen. Aus den Patientendaten wird eine durch eine Positionierung des Patienten auf der Liege erfolgende Liegenbrettverformung errechnet. Anschließend wird die Position der Patientenliege nach Maßgabe der errechneten Liegenbrettverformung für eine zielgerichtete Bestrahlung angepasst. Das Verfahren ermöglicht eine zielgenauere Bestrahlung von Patienten.

FIG 2

EP 1 994 959 A1

**Beschreibung**

Die Erfindung betrifft ein Verfahren und eine Vorrichtung zur automatischen Positionskorrektur einer Patientenliege für eine zielgerichtete Bestrahlung eines Patienten.

In der Medizin ist die Bestrahlung von Patienten mit verschiedenen Techniken (z.B. Röntgenstrahlung, Ultraschall, Ionenstrahlung) zu therapeutischen oder diagnostischen Zwecken üblich. Vor allem bei therapeutischer Bestrahlung (z.B. Gammastrahlen, Teilchen) ist eine möglichst genaue Ausrichtung des Strahls auf das erkrankte Gewebe essentiell für den Behandlungserfolg.

So hängt z.B. der Erfolg einer Tumorbehandlung von der Genauigkeit der Tumorbestrahlung bzw. der Genauigkeit der Strahlenausrichtung auf den Tumor ab. Diese wiederum wird beeinflusst durch die Patientenlagerung sowie die Steifigkeit des verwendeten Patientenlagerungssystems und natürlich dem darauf befestigten Liegenbrett. Die elastischen Verformungen und damit auch die Ungenauigkeiten bei der Positionierung sind näherungsweise direkt proportional zum Patientengewicht. Die Anforderungen bzgl. des zulässigen Patientengewichtes steigen stetig (aktuell 200kg) und damit auch die Steifigkeitsanforderungen an solche Systeme. Ein wesentlicher Bestandteil der Verformungen kommt immer aus dem Liegenbrett. Durch Einsatz hochsteifer Materialien wie CFK (kohlenstofffaserverstärkter Kunststoff) kann zwar eine geringe Durchbiegung erreicht werden, dennoch erreicht diese über das Liegenbrett gesehen Werte von über 10 mm. Gerade für Therapieanwendungen ist dies eine Verformung, die den Therapieerfolg signifikant beeinflussen kann.

Die Erfindung hat zur Aufgabe, eine genauere Bestrahlung von Patienten zu ermöglichen.

Die Aufgabe wird gelöst durch ein Verfahren nach Anspruch 1 bzw. eine Vorrichtung nach Anspruch 9.

Erfindungsgemäß wird eine automatische Positionskorrektur einer Patientenliege für eine zielgerichtete Bestrahlung eines Patienten vorgenommen. Die Positionskorrektur erfolgt nach Maßgabe von Patientendaten. Auf Basis dieser Patientendaten wird eine durch eine Positionierung des Patienten auf der Liege erfolgende Liegenbrettverformung errechnet. Die Position der Patientenliege wird anschließend, z.B. mittels eines Robotersystems, nach Maßgabe der errechneten Liegenbrettverformung für eine zielgerichtete Bestrahlung angepasst.

Durch die Erfindung wird die durch den Patienten hervorgerufene Verformung des Liegenbretts kompensiert. Dadurch wird eine genauere Einstellung des Strahls und damit eine bessere Behandlung (oder eine genauere Diagnose bei diagnostischer Bestrahlung) erzielt.

Das exakte Biegeverhalten der Liege unter Einfluss eines Patienten kann in der Praxis nicht bestimmt werden. Zum einen können in der Situation der Vorbereitung der Bestrahlung nicht alle für die Verformung des Liegenbrettes relevanten Patientendaten (Körperform und Gewichtsverteilung) ermittelt werden. Zum anderen ist eine exakte Berechnung wegen der Unregelmäßigkeit der Körperform und der komplexen Materialeigenschaften des Liegebrettes mit hohem Aufwand verbunden. Daher werden im Rahmen von Weiterbildungen zwei Maßnahmen vorgeschlagen, die die Umsetzung der Erfindung in der Praxis erleichtern.

Gemäß einer ersten Weiterbildung werden als Patientendaten für die Positionskorrektur das Patientengewicht und eine Information über die Lage des Schwerpunkts des Patienten verwendet. Die Information ist z.B. durch den (evtl. auf eine bestimmte Richtung projizierten) Abstand des Schwerpunkts von einem Unterstützungspunkt oder Auflagepunkt gegeben. Bei einer Unterstützungs- bzw. Auflagefläche kann es z.B. der Abstand zu einem zentralen Punkt (z.B. Mittelpunkt) dieser Fläche sein. Diese beiden Informationen erlauben eine vereinfachte Beschreibung der durch den Patienten hervorgerufenen Verbiegung des Liegebretts. Weitere patientenbezogene Informationen können hinzugenommen werden, um diese Beschreibung zu verfeinern.

Eine zweite Weiterbildung sieht vor, die Liegenverbiegung mittels einer empirisch gewonnenen, liegenspezifischen Formel vorzunehmen. Durch die empirische Anpassung der Formel an die Liegeneigenschaften eines Liegentyps kann eine hohe Genauigkeit erreicht werden. Eine analytische Beschreibung wäre alternativ möglich, ist aber vielfach zwecks Komplexitätsreduzierung mit Näherungen verbunden, die Genauigkeitseinbußen mit sich bringen.

Für die Beschreibung weiterer Ausgestaltungen ist es zweckdienlich, von folgendem Koordinatensystem auszugehen. Die x-Achse sei im Wesentlichen parallel zu der Längsrichtung der unbelasteten (d.h. kein Patient bzw. keine Verbiegung) Liege bzw. des unbelasteten Liegenbretts. Im Wesentlichen parallel bedeutet dabei, dass ein dominierender Teil der Liegenoberfläche parallel zur x-Achse ist. Die y-Achse sei parallel zur Querrichtung der unbelasteten Liege und die z-Achse sei orthogonal zu den beiden anderen Achsen (d.h. orthogonal zum Liegenbrett). Dabei soll die Benennung der Achsen nicht und die Angabe des Koordinatensystems nur soweit zur Beschreibung folgender Weiterbildungen erforderlich als einschränkend interpretiert werden.

Gemäß einer Ausgestaltung wird eine Formel verwendet, die die Positionsveränderung in z-Richtung als Funktion von der x-Position, des Patientengewichtes und einer Information über die Lage des Schwerpunkts (z.B. den x-Abstand des Schwerpunkts von einer das Liegenbrett unterstützenden Roboterhand) beschreibt. Diese Funktion kann von weiteren Variablen, z.B. von einer z-Position, abhängen, wenn die für die jeweilige Liegenform dadurch ein signifikanter Genauigkeitsgewinn zu erzielen ist. Zusätzlich kann eine Funktion für eine Positionsveränderung in x-Richtung oder y-Richtung aufgestellt werden, die von denselben Variablen abhängt.

Die Funktion kann aus einer Überlagerung von Ansatzfunktionen gebildet sein, deren Koeffizienten durch eine liegenspezifische Anpassung bzw. einen Fit bestimmt wurden. Mögliche Ansatzfunktionen sind dabei durch Monome, die z.B. zu Polynomen überlagert werden, oder trigonometrische Funktionen.

Die Erfindung beinhaltet auch eine Vorrichtung zur automatischen Positionskorrektur einer Patientenliege für eine zielgerichtete Bestrahlung eines Patienten. Diese umfasst Mittel zur Berechnung einer durch eine Positionierung des Patienten auf der Liege erfolgenden Liegenbrettverformung auf Grundlage von Patientendaten, z.B. Software oder Hardware, und Mittel zur Anpassung der Position der Patientenliege nach Maßgabe der errechneten Liegenbrettverformung für eine zielgerichtete Bestrahlung, z.B. eine Roboterkonstruktion.

Im Folgenden wird der Erfindungsgegenstand anhand eines Ausführungsbeispiels näher erläutert. Es zeigen:

Fig. 1: einen Roboterarm mit mehreren Freiheitsgraden zur Anpassung der Liegenbrettposition

Fig. 2: eine schematische Darstellung der Liegenbrettverformung

Fig. 3: eine schematische Darstellung des Patienten auf dem Liegenbrett

Fig. 4: eine Auswertung von Liegenbrettmessreihen zur Ermittlung einer empirischen Formel für die Liegenbrettverformung

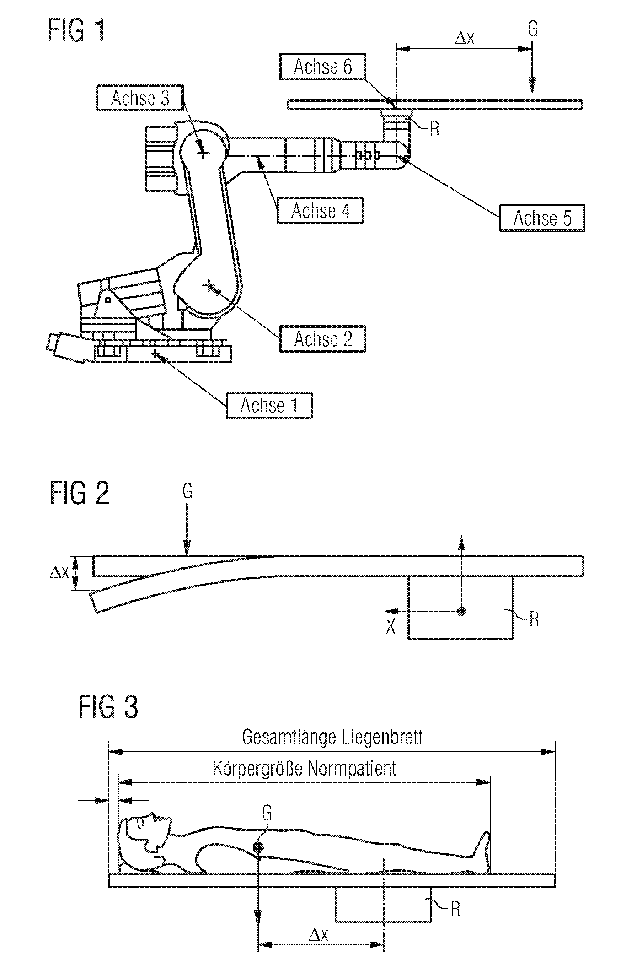

In Fig. 1 ist ein Robotersystem mit einem ein Liegenbrett L tragenden Roboterarm bzw. einer Roboterhand R gezeigt. Diese Roboterhand R kann mittels einer Steuerung (in der Figur nicht dargestellt) die Liegenposition nach Maßgabe eingebbarer Koordinaten einstellen. Die Positionierung eines Patienten auf dem Liegenbrett führt durch dessen Gewicht zu einer Liegenbrettverbiegung. In Fig. 1 ist für einen hypothetischen Beispielfall der Angriffspunkt der Gewichtskraft G bzw. der Schwerpunkt des Patienten eingezeichnet. Dieser Angriffspunkt hat einen Abstand $\Delta x$ von dem Roboterarm bzw. dem Punkt, wo das Liegenbrett mit der Roboterhand R verbunden ist.

Die durch Die Gewichtskraft G des Patienten hervorgerufene Liegenbrettverbiegung ist schematisch in Fig. 2 gezeichnet. Die im Abstand $\Delta x$ von der Roboterhand R (bzw. von dem auf die x-Achse projizierten Mittelpunkt des Verbindungsbereiches von Roboterhand und Liegenbrett) angreifende Gewichtskraft G führt zu einer Liegenbrettverbiegung dz in z-Richtung. Dabei wird das oben eingeführte Koordinatensystem für die Richtungsangeben verwendet.

Die Liegenbrettverbiegung wird zwecks einer präziseren Bestrahlung mittels des in Fig. 1 gezeigten Robotersystems kompensiert. Für diese Kompensation ist erforderlich, die Liegenbrettabweichung dz zumindest näherungsweise zu bestimmen, um diese Größe zur Steuerung der Liegenanpassung durch das Robotersystem zu verwenden. In Fig. 3 ist schematisch ein auf dem Liegenbrett positionierter Patient gezeigt. Typische bei dieser Konstellation ausgezeichnete Größen in der Längsrichtung (x-Richtung) sind die Gesamtlänge des Liegenbretts, die Gesamtlänge des Patienten und der Abstand $\Delta x$ des Patentenschwerpunkts zum Mittelpunkt der Roboterhand R. Die Erfinder haben erkannt, dass das Patientengewicht G und der Abstand $\Delta x$ des Schwerpunkts von der Roboterhand R eine adäquate Beschreibung der Liegenbrettverformung dz ermöglichen.

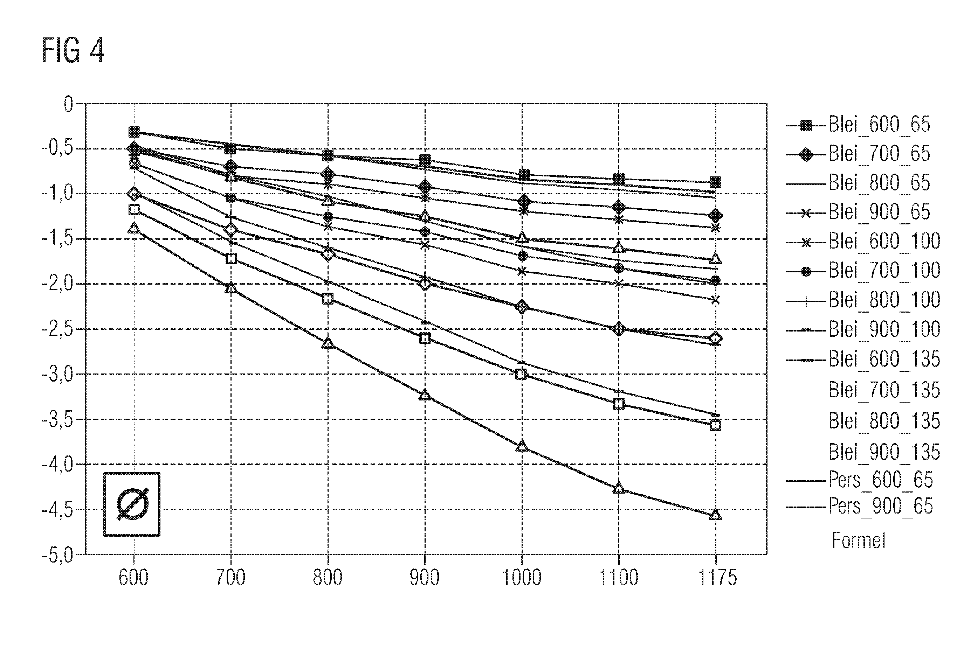

Für die Beschreibung wird eine empirisch ermittelte Formel verwendet. Ausgangspunkt ist ein Ansatz mit Ansatzfunktionen, z.B. ein Polynom n-ten Grades (d.h. $f(x) = \Sigma A_i * x^{**}i$, $1=0..n$) mit geeignet gewähltem n. Andere Ansatzfunktionen als Monome, z.B. trigonometrische Funktionen sind ebenfalls verwendbar. Zur Bestimmung der bei dem Ansatz verwendeten Koeffizienten (z.B. $A_i$) wurden Messreihen durchgeführt, anhand derer das Verformungsverhalten für unterschiedliche Gewichte über den Behandlungsbereich erfasst wurden. Die Formel bzw. die Koeffizienten werden dann an die Messreihen angepasst bzw. gefittet. In Fig. 4 ist die Auswertung einiger Messreihen der Liegenbrettvermessung zu sehen. Bei den Messreihen wurden z.T. Bleigewichte mit verschiedenen Schwerpunktsabständen $\Delta x$ und Gewichten G und t.w. Personen verwendet. Die Notation in der Figur gibt dabei zuerst $\Delta x$ in mm und dann das Gewicht G in kg wieder. Z.B. ist Blei_600_65 eine Messreihe für ein Bleigewicht im Abstand von 600 mm von dem Mittelpunkt der Roboterhand R, wobei das Gewicht 65 kg beträgt. In der Fig. 4 zeigt dabei die x-Achse den Abstand zum Mittelpunkt der Roboterhand und die y-Achse die Liegenbrettverformung dz.

Mit diesem Vorgehen wurden folgende Formel für das betroffene Liegenbrett ermittelt:

```
dz(x) = (0.00000285*(x-500.0)*(x-500.0) - 0.007656*(x-500.0)
- 0.6301)*(G/135.0)*(Δx/900.0)*(Δx/900.0)
```

Bei der Formel müssen die Eingangsgrößen Patientengewicht G und Schwerpunktslage $\Delta x$ eingegeben werden. Die für die Behandlung relevante Liegenbrettverbiegung dz ergibt sich dann durch Eingabe der x-Position der zu bestrahlenden Stelle (betroffenes Gewebe bzw. Tumor). Die Steuerung des Robotersystems greift auf diese Größe dz dann zurück, um die Liegenbrettposition zu korrigieren.

Wie anhand von Fig. 2 überlegt werden kann, gibt es bei der Verbiegung auch ein geringe Abweichung in x-Richtung. Diese kann mit der oben beschriebenen Vorgehensweise ebenfalls kompensiert werden. Je nach Unterstützungspunkt der Liege ist auch eine Abweichung in y-Richtung möglich. In diesem Fall kann entsprechend vorgegangen werden.

Eine der Erfindungsmeldung zugrunde liegende Idee ist es, das Liegenbrett mathematisch so zu erfassen, dass die Durchbiegung berechnet und vom Robotersystem bzw. Patient Handling System (PHS) kompensiert werden kann. Dies geschieht im Zuge des Ausführungsbeispiels in Abhängigkeit von den Größen "Patientengewicht" und "Patientenschwerpunktlage". Anhand dieser Variablen kann dann für eine bestimmte Position auf dem Liegenbrett die Durchbiegung und deren Auswirkung auf die Tumorlage berechnet werden. Andere Ausgestaltungen sind dem Fachmann aus dem beschriebenen konzeptionellen Vorgehen unmittelbar ersichtlich.

**Patentansprüche**

1. Verfahren zur automatischen Positionskorrektur einer Patientenliege für eine zielgerichtete Bestrahlung eines Patienten, bei dem

    - die Positionskorrektur nach Maßgabe von Patientendaten vorgenommen wird,
    - aus den Patientendaten eine durch eine Positionierung des Patienten auf der Liege erfolgende Liegenbrettverformung errechnet wird, und
    - die Position der Patientenliege nach Maßgabe der errechneten Liegenbrettverformung für eine zielgerichtete Bestrahlung angepasst wird.

2. Verfahren nach Anspruch 1,
   **dadurch gekennzeichnet, dass**

    - als Patientendaten für die Positionskorrektur das Patientengewicht und eine Information über die Lage des Schwerpunkts des Patienten verwendet werden.

3. Verfahren nach einem der vorhergehenden Ansprüche,
   **dadurch gekennzeichnet, dass**
   die Errechnung mit einer empirisch gewonnenen Formel vorgenommen wird.

4. Verfahren nach einem der vorhergehenden Ansprüche,
   **dadurch gekennzeichnet, dass**

    - ein Koordinatensystem gegeben ist, dessen x-Achse im Wesentlichen parallel zu der unbelasteten Längsrichtung der Liegenoberfläche ist, dessen y-Achse parallel der Querrichtung der Liegenoberfläche und dessen z-Achse orthogonal zu den beiden anderen Achsen ist.

5. Verfahren nach Anspruch 4,
   **dadurch gekennzeichnet, dass**
   eine Formel verwendet wird, die die Positionsveränderung in z-Richtung als Funktion von der x-Position, des Patientengewichtes und einer Information über die Lage des Schwerpunkts beschreibt.

6. Verfahren nach Anspruch 5,
   **dadurch gekennzeichnet, dass**
   die Information über die Lage des Schwerpunkts durch den x-Abstand des Schwerpunkts von einem Unterstützungspunkt oder Auflagepunkt der Liege gegeben ist.

7. Verfahren nach Anspruch 5 oder 6,
   **dadurch gekennzeichnet, dass**
   die Funktion aus einer Überlagerung von Ansatzfunktionen gebildet ist, deren Koeffizienten durch eine liegenspezifische Anpassung bestimmt wurden.

8. Verfahren nach einem der vorhergehenden Ansprüche,
   **dadurch gekennzeichnet, dass**

die Anpassung der Patientenliege mittels eines Robotersystems erfolgt.

9. Vorrichtung zur automatischen Positionskorrektur einer Patientenliege für eine zielgerichtete Bestrahlung eines Patienten,

   - mit Mitteln zur Berechnung einer durch eine Positionierung des Patienten auf der Liege erfolgenden Liegenbrettverformung auf Grundlage von Patientendaten, und
   - Mittel zur Anpassung der Position der Patientenliege nach Maßgabe der errechneten Liegenbrettverformung für eine zielgerichtete Bestrahlung.

10. Vorrichtung nach Anspruch 9,
    **dadurch gekennzeichnet, dass**
    die Mittel zur Anpassung der Position ein Robotersystem umfasst, durch die die Position des Liegenbretts veränderbar ist.

11. Vorrichtung nach Anspruch 9 oder 10,
    **dadurch gekennzeichnet, dass**
    die Mittel zur Durchführung eines Verfahrens nach einem der Ansprüche 1 bis 8 ausgestaltet sind.

## FIG 1

Achse 6  Achse 3  Achse 4  Achse 5  Achse 2  Achse 1  $\Delta x$  G  R

## FIG 2

G  $\Delta x$  X  R

## FIG 3

Gesamtlänge Liegenbrett
Körpergröße Normpatient
G  R  $\Delta x$

FIG 4

Blei_600_65
Blei_700_65
Blei_800_65
Blei_900_65
Blei_600_100
Blei_700_100
Blei_800_100
Blei_900_100
Blei_600_135
Blei_700_135
Blei_800_135
Blei_900_135
Pers_600_65
Pers_900_65
Formel

**Europäisches Patentamt**

**EUROPÄISCHER RECHERCHENBERICHT**

Nummer der Anmeldung

EP 08 10 3700

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 103 25 301 A1 (SIEMENS AG [DE]) 5. Januar 2005 (2005-01-05) * Absätze [0007] - [0019]; Abbildung 1 * | 1,2,4, 8-11 | INV. A61N5/10 |
| Y | | 3 | |
| A | | 5-7 | |
| | ----- | | |
| Y | US 2005/234327 A1 (SARACEN MICHAEL J [US] ET AL) 20. Oktober 2005 (2005-10-20) * Absätze [0033] - [0036], [0085]; Abbildungen 2A,2F * | 3 | |
| | ----- | | |
| Y | WO 2006/083703 A (INDIANA UNIVERSITY RES & TECHN [US]; TIMMERMAN ROBERT [US]; PAPIEZ LEC) 10. August 2006 (2006-08-10) * das ganze Dokument * | 3 | |
| | ----- | | |
| A | US 6 094 760 A (NONAKA HIDEKI [JP] ET AL) 1. August 2000 (2000-08-01) * das ganze Dokument * | 1-11 | |
| | ----- | | |
| A | DE 10 2004 061591 B3 (SIEMENS AG [DE]) 3. August 2006 (2006-08-03) * das ganze Dokument * | 1-11 | RECHERCHIERTE SACHGEBIETE (IPC) |
| | ----- | | A61N |

Der vorliegende Recherchenbericht wurde für alle Patentansprüche erstellt

| Recherchenort | Abschlußdatum der Recherche | Prüfer |
|---|---|---|
| München | 11. August 2008 | Link, Tatiana |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

.............................................................................

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 08 10 3700

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

11-08-2008

| Im Recherchenbericht angeführtes Patentdokument | Datum der Veröffentlichung | Mitglied(er) der Patentfamilie | Datum der Veröffentlichung |
|---|---|---|---|
| DE 10325301 A1 | 05-01-2005 | CN 1572256 A<br>US 2004261176 A1 | 02-02-2005<br>30-12-2004 |
| US 2005234327 A1 | 20-10-2005 | WO 2006124434 A2 | 23-11-2006 |
| WO 2006083703 A | 10-08-2006 | CA 2595751 A1 | 03-08-2006 |
| US 6094760 A | 01-08-2000 | BE 1012534 A3 | 05-12-2000 |
| DE 102004061591 B3 | 03-08-2006 | CN 1792331 A<br>JP 2006175236 A<br>US 2006184012 A1 | 28-06-2006<br>06-07-2006<br>17-08-2006 |

EPO FORM P0461

Für nähere Einzelheiten zu diesem Anhang : siehe Amtsblatt des Europäischen Patentamts, Nr.12/82